# EUROPEAN PATENT SPECIFICATION

(11) **EP 2 866 777 B1**
(45) Date of publication and mention of the grant of the patent: **28.02.2018**
(21) Application number: 13732192.3
(22) Date of filing: 28.06.2013
(51) Int. Cl.: A61K 8/34, A61K 8/39, A61K 8/41, A61K 8/46, A61Q 5/04

(54) **REDUCING COMPOSITION COMPRISING AT LEAST ONE SULFUR-CONTAINING REDUCING AGENT, AT LEAST ONE FATTY SUBSTANCE, AT LEAST ONE CATIONIC SURFACTANT AND AT LEAST ONE OXYETHYLENATED NONIONIC SURFACTANT**
REDUZIERENDE ZUSAMMENSETZUNG MIT MINDESTENS EINEM SCHWEFELHALTIGEN REDUKTIONSMITTEL, MINDESTENS EINEM FETTSTOFF, MINDESTENS EINEM KATIONISCHEN TENSID UND MINDESTENS EINEM OXYETHYLIERTEN NICHTIONISCHEN TENSID
COMPOSITION RÉDUCTRICE COMPRENANT AU MOINS UN RÉDUCTEUR CONTENANT DU SOUFRE, AU MOINS UNE SUBSTANCE GRASSE, AU MOINS UN TENSIOACTIF CATIONIQUE ET AU MOINS UN TENSIOACTIF NON IONIQUE OXYÉTHYLÉNÉ

(30) Priority: 29.06.2012 FR 1256263; 31.08.2012 US 201261695335 P
(43) Date of publication of application: 06.05.2015
(73) Proprietor: L'OREAL, 75008 Paris (FR)
(72) Inventor: SIMONET, Frédéric, F-92110 Clichy (FR); LIVOREIL-DJAMPOU, Aude, F-75015 Paris (FR)
(74) Representative: Casalonga
(86) International application number: PCT/EP2013/063709
(87) International publication number: WO 2014/001540

(56) References cited:
- JP-A- 2008 127 380
- US-A1- 2008 025 937
- US-A1- 2008 075 681

## Description

The present invention relates to a reducing cosmetic composition comprising one or more sulfur-containing reducing agents, preferably thiol agents, one or more fatty substances solid at room temperature and with a total amount of more than 4% by weight, one or more cationic surfactants, ammonium bicarbonate and one or more oxyethylenated nonionic surfactants having an ethylene oxide number of from 10 to 25, with a weight ratio of said fatty substance or substances to said cationic surfactant or surfactants of less than or equal to 2.5.

The invention also relates to a method for permanent deformation of keratinous fibres, more particularly of human keratinous fibres such as the hair, which comprises the use on said fibres of the reducing composition defined above and, optionally, of an oxidizing composition.

Many people are dissatisfied with the appearance of their hair, especially those people who have curly hair and usually wish to acquire smooth hair, and, conversely, those people who have straight hair and wish to have curly hair.

One of the techniques commonly used in order to obtain permanent deformation of the hair involves a two-step procedure, the first step of which is to reduce the disulfide bridges present in the keratinous fibre, using a composition containing a suitable reducing agent (reduction step). Once these disulfide bridges have been reduced, the hair is then shaped in the desired manner. This shaping step may involve frizzing the hair or else smoothing it, the result being dependent on the means employed to tension the hair and on the nature of the original keratinous fibres. This tensioning operation may be carried out before, during or after the application of the reducing composition to the hair. Once this first step has been performed, an oxidation step is necessary in order to recreate the disulfide bridges and to stabilize the shape obtained. This operation is commonly carried out using an oxidizing composition (in a step also called the setting step).

This technique therefore allows the hair to be waved (perming procedure) and/or smoothed (straightening). The new shape imposed on the hair by a chemical treatment as stated above is durable over time and resistant particularly to the action of washing with water or using shampoos, as opposed to simple, conventional techniques of temporary deformation, such as setting.

The reducing compositions intended for smoothing or perming of the hair generally include reducing agents in the form of sulfites, bisulfites, alkylphosphines or, preferably, thiols. These reducing compositions are generally in the form of a thickened or unthickened lotion, a cream or a gel.

These compositions may also comprise alkaline agents.

The majority of the cream vehicles used for manufacturing reducing compositions are formulated on the basis of crystallizable compounds, such as fatty alcohols, for example, especially cetylstearyl alcohol, or fatty acid esters, for example cetyl palmitate. These compounds, which are solid at room temperature, are generally melted and then introduced in aqueous phase and emulsified in the hot state. The emulsion is subsequently cooled, thus causing crystallization of these compounds in the form of solid particles of micro-scale size, in other words in the form of crystals. In order to realize the emulsifying step, ionic and/or nonionic surfactants are generally introduced into the formulations. The role of these surfactants is to make the emulsion finer and to produce a smooth and creamy dispersion with no lumps.

Generally speaking, subsequent to the addition of the reducing agents or alkaline agents, it is found that these vehicles have an unsatisfactory texture. More specifically, following this addition, it is observed that creams are obtained that have an excessively fluid texture, possibly resembling that of a milk, or having a disparate and flocculated appearance which is unacceptable in terms of product quality. In other words, the resulting reducing compositions do not have satisfactory texture, especially after the addition of reducing agents or alkaline agents.

Moreover, vehicles of this kind may have uncertain stability in preservation tests, with problems of loss of viscosity or of phase separation/bodying.

In particular, US 2008/0075681 discloses a composition for permanent hair shaping comprising about 0.5% y weight o about 35% by weight of a N-alkyl-2-mercaptoacetamide, about 0.1% by weight to about 30% by weight of at least one hair swelling and penetration enhancing substance and about 5% by weight to about 95% by weight of water.

Especially, this document describes a gel composition in which the weight ratio between solid fatty substances at room temperature and the cationic surfactants is higher than 2.5.

There is therefore a real need to employ reducing compositions, intended for use during a method for permanent deformation of keratinous fibres, which are devoid of all of the drawbacks described above, in other words having satisfactory texture, without the need to increase the level of non-oxyalkylenated fatty substances, and which are stable in storage.

The applicant has found, surprisingly, that it was possible for the desired properties to be obtained by combination, in a reducing composition, of one or more sulfur-containing reducing agents as described below, one or more fatty substances solid at room temperature and present in an amount of more than 4 wt%, one or more cationic surfactants, ammonium bicarbonate, and one or more oxyethylenated nonionic surfactants having an ethylene oxide unit number of from 10 to 25 as described below, the fatty substance(s) and the cationic surfactant(s) being formulated in a weight ratio of less than or equal to 2.5.

The combination defined above, indeed, allows reducing compositions to be obtained that have a satisfactory texture and are homogeneous throughout the manufacturing process and at the time of being applied to keratinous fibres. It is found especially that the reducing compositions exhibit a smooth and uniform texture throughout their manufacture, including at the end, after the addition of reducing agents and of alkaline agents. It is also found that the viscosity of the compositions remains constant during the manufacturing process.

Furthermore, the above-defined combination also allows the reducing compositions obtained to be stable on the shelf, in the presence of reducing agents or alkaline agents, these compositions being more particularly those which are stable on storage at room temperature (25°C) or at temperatures of up to 45°C.

By "stable" in the sense of the present invention is meant that the visual appearance, the texture and the viscosity of the reducing compositions does not change substantially over the course of time under conditions of storage at room temperature or at temperatures less than or equal to 45°C, for a number of days after their manufacture.

In other words, it is found that the viscosity and texture of the reducing compositions is not adversely affected over time, and that the problems of phase separation or of bodying are minimized, or even suppressed, in a satisfactory way.

The reducing compositions may be used in a permanent deformation process, especially for smoothing or perming keratinous fibres, in a satisfactory way, while endowing said fibres with good cosmetic properties.

Accordingly, the reducing compositions have enhanced physicochemical properties and may result in good cosmetic properties when they are used in a method for permanent deformation of keratinous fibres.

The present invention accordingly provides in particular a reducing composition comprising:
- one or more sulfur-containing reducing agents selected from thioglycolic acid, thiolactic acid, acid, 3-mercaptopropionic acid, thiosalicylic acid, thiopropionic acid, and salts thereof,

- one or more fatty substances solid at room temperature and present in a total amount of greater than or equal to 4 wt%, relative to the total weight of the composition,
- one or more cationic surfactants,
- one or more oxyethylenated nonionic surfactants having an ethylene oxide unit number of from 10 to 25 selected from oxyethylenated saturated fatty alcohols, the weight ratio of the solid fatty substance(s) to the cationic surfactant(s) being less than or equal to 2.5, and
- ammonium bicarbonate.

The reducing composition according to the invention spreads easily over the keratinous fibres, thereby facilitating its application in the course of a method for permanent deformation of the keratinous fibres, and can be rinsed off easily.

The reducing composition according to the invention is present preferably in the form of a cream for the treatment of keratinous fibres, more particularly of human keratinous fibres such as the hair.

The invention further, accordingly, provides a method for permanent deformation, in particular for smoothing and for permanent waving of keratinous fibres, more particularly of human keratinous fibres such as the hair, which comprises:
- a step of applying to said fibres a reducing composition as defined above, and the time sufficient for shaping is allowed to elapse, and
- optionally, a step of applying to said fibres an oxidizing composition for a time sufficient for the fixing of the shape.

The method for permanent deformation allows the keratinous fibres to be smoothed or waved, while endowing them with good cosmetic properties.

Further objects, features, aspects and advantages of the invention will emerge more clearly from the reading of the description and examples which follow.

Further objects, features, aspects and advantages of the invention will emerge more clearly from the reading of the description and examples which follow.

Thiol reducing agents which can be used in the reducing composition include thiol reducing agents selected from thioglycolic acid, thiolactic acid, thiomalic acid, 2-mercaptopropionic acid, 3-mercaptopropionic acid, thiosalicylic acid, thiopropionic acid and salts thereof.

The sulfur-containing reducing agent or agents used in the reducing composition are thiol reducing agents, more particularly thioglycolic acid and thiolactic acid or salts thereof, especially alkali metal, alkaline-earth metal or ammonium salts, cysteine, and mixtures thereof.

The sulfur-containing reducing agent used in the reducing composition is preferably thioglycolic acid and its salts, especially ammonium thioglycolate.

The sulfur-containing reducing agent or agents may be present in the composition according to the invention in an amount of from 0.1 to 50 wt%, preferably in an amount of from 1 to 20 wt%, relative to the total weight of the reducing composition.

As indicated above, the reducing composition comprises one or more fatty substances solid at room temperature.

A fatty substance in the sense of the present invention is an organic compound which is insoluble in water at ordinary room temperature (20-25°C) and at atmospheric pressure (760 mm Hg, or 1.013 x 10⁵ Pa), having a solubility in water of less than 5%, preferably of less than 1% and even more preferably of less than 0.1%. The fatty substances generally include in their structure a hydrocarbon chain containing at least 6 carbon atoms. In addition, the fatty substances are generally soluble in organic solvents under the same temperature and pressure conditions, for instance chloroform, ethanol, benzene, liquid petrolatum or decamethylcyclopentasiloxane.

The fatty substances, furthermore, are not (poly)oxyalkylenated and not (poly)glycerolated. In other words, the fatty substances do not include in their structure an ethylene oxide or glycerol or propylene glycol unit.

By solid fatty substance is meant, in the sense of the present invention, a fatty substance which is not liquid at room temperature (20-25°C) and at atmospheric pressure (760 mm Hg, or 1.013 x 10⁵ Pa), more particularly a compound which is solid or a compound which has a viscosity of greater than 2 Pa.s at a shear rate of 1s⁻¹ under the aforementioned conditions.

The solid fatty substances used in the reducing composition in accordance with the invention have a melting temperature of greater than room temperature, preferably a melting temperature of greater than or equal to 40°C, more preferably from 46 to 95°C.

The fatty substances solid at room temperature may be selected from fatty alcohols, fatty acid esters and/or fatty alcohol esters, mineral, vegetable or animal waxes, fatty amides, silicones, solid fatty ethers and mixtures thereof.

Solid fatty alcohols suitable for the implementation of the invention are selected more particularly from alcohols conforming to the formula (I) below.

R¹-OH (I)

where R¹ denotes a saturated linear alkyl radical having from 16 to 30 carbon atoms. Examples include cetyl alcohol, stearyl alcohol and a mixture thereof (cetylstearyl alcohol). More particularly, the non-liquid fatty alcohol is cetylstearyl alcohol.

The non-liquid fatty acid esters and/or fatty alcohol esters include, in particular, solid esters obtained from C₉-C₂₆ fatty acids and from C₉-C₂₆ fatty alcohols.

These esters include octyldodecyl behenate, isocetyl behenate, cetyl lactate, stearyl octanoate, octyl octanoate, cetyl octanoate, decyl oleate, myristyl stearate, octyl palmitate, cetyl palmitate, octyl pelargonate, octyl stearate, alkyl myristates such as cetyl, myristyl or stearyl myristate, and hexyl stearate.

The wax or waxes are preferably non-silicone waxes and are selected in particular from mineral waxes such as paraffin wax, ozokerite, vegetable waxes such as olive wax, rice wax, hydrogenated jojoba wax, carnauba wax, candelilla wax and alfa wax, or absolute flower waxes such as essential wax of blackcurrant flower, sold by Bertin (France), animal waxes such as beeswax or modified beeswaxes (cerabellina), and ceramides.

Solid amides that may be mentioned include ceramides. Ceramides or ceramide analogues, such as the glycoceramides which can be used in the compositions according to the invention, are known per se and are natural or synthetic molecules which may conform to the general formula (II) below: 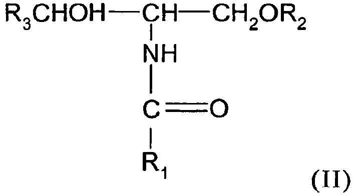 wherein:
- R₁ denotes a saturated or unsaturated, linear or branched akyl radical derived from C₁₄-C₃₀ fatty acids, it being possible for this radical to be substituted by a hydroxyl group in alpha position, or a hydroxyl group in omega position esterified by a saturated or unsaturated C₁₆-C₃₀ fatty acid;
- R₂ denotes a hydrogen atom or a (glycosyl)n, (galactosyl)m or sulfogalactosyl radical, in which n is an integer from 1 to 4 and m is an integer from 1 to 8;
- R₃ denotes a C₁₅-C₂₆ hydrocarbon radical which is saturated or unsaturated in alpha position, it being possible for this radical to be substituted by one or more C₁-C₁₄ alkyl radicals;
with the proviso that in the case of natural ceramides or glycoceramides, R₃ may also denote a C₁₅-C₂₆ alpha-hydroxyalkyl radical, the hydroxyl group being optionally esterified by a C₁₆-C₃₀ alpha-hydroxy acid.

Ceramides preferred in the context of the present invention are those described by Downing in Arch. Dermatol., Vol. 123, 1381-1384, 1987, or those described in French patent FR 2 673 179.

The ceramide or ceramides more particularly preferred according to the invention are the compounds for which R₁ denotes a saturated or unsaturated alkyl derived from C₁₆-C₂₂ fatty acids, R₂ denotes a hydrogen atom, and R₃ denotes a saturated C₁₅ linear radical.

Such compounds are, for example:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
or mixtures of these compounds.

More preferably still, ceramides are used for which R₁ denotes a saturated or unsaturated alkyl radical derived from fatty acids, R₂ denotes a galactosyl or sulfogalactosyl radical, and R₃ denotes a -CH=CH-(CH₂)₁₂-CH₃ group.

Other waxes or waxy raw materials that can be used according to the invention are, in particular, polyethylene waxes or polyolefin waxes in general.

The solid silicones in accordance with the invention may take the form of waxes or of resins. They may be organically modified.

The organopolysiloxane resins which can be used in accordance with the invention are crosslinked siloxane systems containing the following units:

R₂SiO_{2/2}, R₃SiO_{1/2}, RSiO_{3/2} and SiO_{4/2}

in which R represents an alkyl possessing 1 to 16 carbon atoms. Among these products, those that are particularly preferred are those in which R denotes a C₁-C₄ lower alkyl group, more particularly methyl.

Mention may be made, among these resins, of the product sold under the name Dow Corning 593 or those sold under the names Silicone Fluid SS 4230 and SS 4267 by the company General Electric, which are silicones of dimethyl/trimethylsiloxane structure.

Mention may also be made of the resins of the trimethylsiloxysilicate type, sold in particular under the names X22-4914, X21-5034 and X21-5037 by the company Shin-Etsu.

Organically modified silicones include polyorganosiloxanes containing alkoxy groups, such as the product sold under the name Abil Wax® 2428, 2434 and 2440 by the company Goldschmidt.

The non-liquid fatty ethers are selected from dialkyl ethers and in particular dicetyl ether and distearyl ether, alone or as a mixture.

The fatty substances solid at room temperature that are used in the cosmetic composition according to the invention are preferably selected from fatty alcohols, esters of fatty acids and of fatty alcohols, and mixtures thereof.

The fatty substances are more preferably selected from cetyl alcohol, stearyl alcohol, cetylstearyl alcohol, cetyl palmitate, and mixtures thereof.

More preferably still, the solid fatty substances are selected from linear, saturated fatty alcohols containing from 12 to 30 carbon atoms, and more particularly from cetyl alcohol, stearyl alcohol and mixtures thereof.

The fatty substance or substances solid at room temperature are present in the reducing composition in accordance with the present invention in a total amount greater than or equal to 4 wt%, relative to the total weight of the composition, preferably in a total amount of from 4 wt% to 30 wt% and more preferably in a total amount of between 4.5 wt% and 20 wt%, relative to the total weight of the reducing composition.

As indicated above, the reducing composition further comprises one or more cationic surfactants.

Cationic surfactants used in the reducing composition may be selected from salts of optionally polyoxyalkylenated primary, secondary or tertiary fatty amines, quaternary ammonium salts, and mixtures thereof.

The cationic surfactants used in the reducing composition are preferably quaternary ammonium salts.

Quaternary ammonium salts include especially, for example:
- those corresponding to the general formula (III) below: 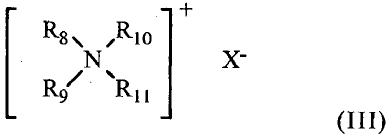 in which the radicals R₈ to R₁₁, which may be identical or different, represent a linear or branched aliphatic radical comprising from 1 to 30 carbon atoms, or an aromatic radical such as aryl or alkylaryl, with at least one of the radicals R₈ to R₁₁ denoting a linear or branched alkyl radical comprising from 10 to 30 carbon atoms, and X- denoting an organic or inorganic anion. The aliphatic radicals may include heteroatoms such as, in particular, oxygen, nitrogen, sulfur and halogens. The aliphatic radicals are selected for example from C₁₋₃₀ alkyl, C₁₋₃₀ alkoxy, polyoxyalkylene (C₂-C₆), C₁₋₃₀ alkylamide, alkyl(C₁₂-C₂₂)amidoalkyl(C₂-C₆) and C₁₋₃₀ hydroxyalkyl radicals; X is an anion selected from the group of halides, phosphates, acetates, lactates, alkyl(C₂-C₆)sulfates and alkyl- or alkylaryl-sulfonates.
   Among the quaternary ammonium salts of formula (III), preference is firstly given to tetraalkylammonium chlorides such as, for example, dialkyldimethylammonium or alkyltrimethylammonium chlorides in which the alkyl radical comprises approximately from 12 to 22 carbon atoms, more particularly behenyltrimethylammonium, distearyldimethylammonium, cetyltrimethylammonium, and benzyl-dimethylstearylammonium chlorides, or else, secondly, to palmitylamidopropyltrimethylammonium chloride or stearamidopropyldimethyl(myristyl acetate)-ammonium chloride, which is sold under the name Ceraphyl® 70 by the company Van Dyk.
- quaternary ammonium salts of imidazoline, such as, for example, those of formula (IV) below: 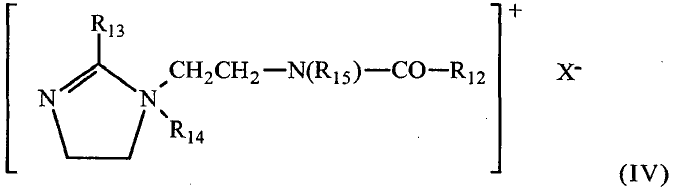 in which R₁₂ represents an alkenyl or alkyl radical comprising from 8 to 30 carbon atoms, derived for example from tallow fatty acids, R₁₃ represents a hydrogen atom, a C₁-C₄ alkyl radical or an alkenyl or alkyl radical comprising from 8 to 30 carbon atoms, R₁₄ represents a C₁-C₄ alkyl radical, R₁₅ represents a hydrogen atom or a C₁-C₄ alkyl radical, X⁻ is an anion selected from the group of halides, phosphates, acetates, lactates, alkyl sulfates and alkyl- or alkylaryl-sulfonates. Preferably, R₁₂ and R₁₃ denote a mixture of alkenyl or alkyl radicals comprising from 12 to 21 carbon atoms, for example derived from tallow fatty acids, R₁₄ denotes a methyl radical and R₁₅ denotes a hydrogen atom. A product of this kind is sold for example under the name Rewoquat® W 75 by the company Rewo;
- quaternary diammonium or triammonium salts, particularly of formula (V) below: 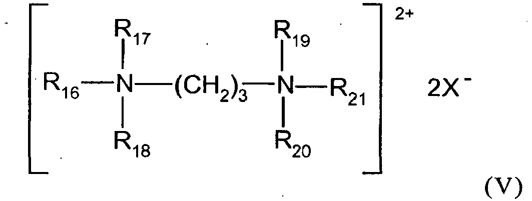 in which formula (V):
   R₁₆ denotes an alkyl group comprising approximately from 16 to 30 carbon atoms, which is optionally hydroxylated and/or interrupted with one or more oxygen atoms;
   R₁₇ is selected from hydrogen, an alkyl group comprising from 1 to 4 carbon atoms or a group -(CH₂)₃-N⁺(R₁₆ₐ)(R₁₇ₐ)(R₁₈ₐ), X⁻;
   R₁₆ₐ, R₁₇ₐ, R₁₈ₐ, R₁₈, R₁₉, R₂₀ and R₂₁, which may be identical or different, are selected from hydrogen and an alkyl group comprising from 1 to 4 carbon atoms; and
   X⁻, which may be identical or different, represent an organic or inorganic anionic counterion, such as that selected from halides, acetates, phosphates, nitrates, alkyl(C₁-C₄) sulfates, alkyl(C₁-C₄)- or alkyl(C₁-C₄)aryl-sulfonates, more particularly methyl sulfate and ethyl sulfate.

Compounds of this kind are, for example, Finquat CT-P, available from the company Finetex (Quaternium 89), and Finquat CT, available from the company Finetex (Quaternium 75);
- -quaternary ammonium salts containing one or more ester functions, such as those of formula (VI) below: 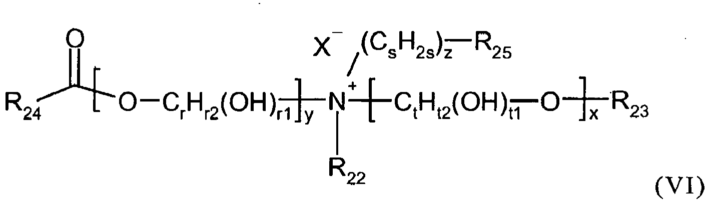 in which formula (VI):
   R₂₂ is selected from C₁-C₆ alkyl groups and C₁-C₆ hydroxyalkyl or C₁-C₆ dihydroxyalkyl groups,
   R₂₃ is selected from:
      - the group 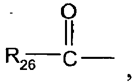
      - linear or branched, saturated or unsaturated C₁-C₂₂ hydrocarbon groups R₂₇,
      - hydrogen atom,
   R₂₅ is selected from:
      - the group 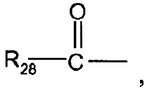
      - linear or branched, saturated or unsaturated C₁-C₆ hydrocarbon groups R₂₉,
      - hydrogen atom,
   R₂₄, R₂₆ and R₂₈, which are identical or different, are selected from linear or branched, saturated or unsaturated C₇-C₂₁ hydrocarbon groups;
   r, s and t, which are identical or different, are integers from 2 to 6,
   rl and t1, which are identical or different, are equal to 0 or 1, with r2+r1=2r and t1+t2=2t,
   y is an integer ranging from 1 to 10,
   x and z, which are identical or different, are integers from 0 to 10,
   X⁻ represents an organic or inorganic anionic counterion,
with the proviso that the sum x + y + z equals from 1 to 15, that, when x is 0, then R₂₃ denotes R₂₇ and that, when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z has a value from 1 to 10.

When R₂₃ is an R₂₇ hydrocarbon group, it may be long and may have from 12 to 22 carbon atoms, or may be short and may have from 1 to 3 carbon atoms.

When R₂₅ is an R₂₉ hydrocarbon group, it preferably has 1 to 3 carbon atoms.

Advantageously, R₂₄, R₂₆ and R₂₈, which are identical or different, are selected from linear or branched, saturated or unsaturated C₁₁-C₂₁ hydrocarbon groups, and more particularly from linear or branched, saturated or unsaturated C₁₁-C₂₁ alkyl and alkenyl groups.

Preferably, x and z, which are identical or different, are equal to 0 or 1.

Advantageously, y is 1.

Preferably, r, s and t, which are identical or different, are 2 or 3, and more particularly they are 2.

The anionic counterion X⁻ is preferably a halide, such as chloride, bromide or iodide; a alkyl(C₁-C₄) sulfate or a alkyl(C₁-C₄)-or alkyl(C₁-C₄)aryl-sulfonate. It is possible, however, to use methanesulfonate, phosphate, nitrate or tosylate, an anion derived from organic acid such as acetate or lactate, or any other anion compatible with the ester-functional ammonium.

The anionic counterion X⁻ is even more particularly chloride, methyl sulfate or ethyl sulfate.

Use is made more particularly, in the composition according to the invention, of the ammonium salts of formula (VI) in which:
- R₂₂ denotes a methyl or ethyl group,
- x and y are equal to 1,
- z is equal to 0 or 1,
- r, s and t are equal to 2,
- R₂₃ is selected from: the group 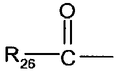 methyl, ethyl or C₁₄-C₂₂ hydrocarbon groups,
   a hydrogen atom,
- R₂₅ is selected from:
   the group 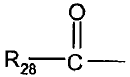 a hydrogen atom,
- R₂₄, R₂₆ and R₂₈, which may be identical or different, are selected from linear or branched, saturated or unsaturated C₁₃-C₁₇ hydrocarbon groups, and preferably from linear or branched, saturated or unsaturated C₁₃-C₁₇ alkyl and alkenyl groups.

Advantageously, the hydrocarbon radicals are linear.

Mention may be made, for example, among the compounds of formula (VI), of diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts, especially the chloride or the methyl sulfate, and mixtures of these compounds. The acyl groups preferably have 14 to 18 carbon atoms and originate more particularly from a vegetable oil such as palm oil or sunflower oil. When the compound contains a plurality of acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca, Rewoquat® WE 18 by the company Rewo-Witco.

The composition according to the invention may contain, for example, a mixture of quaternary ammonium monoester, diester and triester salts, with a majority by weight of diester salts.

It is also possible to use the ammonium salts containing at least one ester function that are described in patents US-A-4874554 and US-A-4137180.

Use may be made of the behenoylhydroxypropyltrimethylammonium chloride available from Kao under the name Quatarmin BTC 131.

Preferably, the ammonium salts containing at least one ester function contain two ester functions.

Among the quaternary ammonium salts containing at least one ester function that can be used, it is preferred to use dipalmitoylethylhydroxyethylmethylammonium salts.

The cationic surfactants used in the reducing composition are preferably selected from quaternary ammonium salts of formula (III) and quaternary ammonium salts comprising at least one ester function, especially those selected from those corresponding to the formula (VI).

The cationic surfactants used in the composition are preferably selected from behenyltrimethylammonium, distearyldimethylammonium, cetyltrimethylammonium and benzyldimethylstearylammonium chlorides, or else from palmitylamidopropyltrimethylammonium or stearamidopropyldimethyl(myristyl acetate)-ammonium chlorides. ethylene oxide unit number of from 10 to 25 selected from oxyethylenated saturated fatty alcohols.

Examples of oxyethylenated nonionic surfactants include the following:
oxyethylenated, linear or branched, saturated C₈-C₃₀, preferably C₁₂-C₂₂, fatty alcohols.

The oxyethylenated nonionic surfactants are selected from oxyethylenated fatty alcohols.

A fatty alcohol for the purposes of the present invention is an alcohol comprising at least six carbon atoms in its structure.

The oxyethylenated nonionic surfactants are selected more particularly from oxyethylenated fatty alcohols in which the fatty chain of the fatty alcohol is a C₈-C₃₀ more particularly C₁₀-C₂₂, chain, these oxyethylenated alcohols being saturated, linear or branched, and having an ethylene oxide unit number of from 10 to 25, such as, for example, the addition products of ethylene oxide with lauryl alcohol, containing from 10 to 25 oxyethylene groups (for example Laureth-10, Laureth 11, Laureth-12, Laureth-13, Laureth-14, Laureth-15, Laureth-16, Laureth-20, Laureth-21, Laureth-23, Laureth-25 in CTFA nomenclature), more particularly 10 oxyethylene groups (Laureth-10 in CTFA nomenclature); addition products of ethylene oxide with behenyl alcohol, comprising from 10 to 25 oxyethylene groups (for example Beheneth-10, Beheneth-20 to Beheneth-25 in CTFA nomenclature); addition products of ethylene oxide with cetearyl alcohol (a mixtutre of cetyl alcohol and stearyl alcohol), comprising from 10 to 25 oxyethylene groups (for example Ceteareth-10, Ceteareth-11, Ceteareth-12, Ceteareth-13, Ceteareth-14, Ceteareth-15, Ceteareth-16, Ceteareth-17, Ceteareth-18, Ceteareth-20, Ceteareth-22, Ceteareth-23, Ceteareth-24, Ceteareth-25 in CTFA nomenclature); addition products of ethylene oxide with cetyl alcohol, comprising from 10 to 25 oxyethylene groups (for example Ceteth-10, Ceteth-12, Ceteth-14, Ceteth-15, Ceteth-16, Ceteth-17, Ceteth-18, Ceteth-20, Ceteth-23, Ceteth-24, Ceteth-25 in CTFA nomenclature); addition products of ethylene oxide with stearyl alcohol, comprising from 10 to 25 oxyethylene groups (for example Steareth-10, Steareth-11, Steareth-13, Steareth-14, Steareth-15, Steareth-16, Steareth-20, Steareth-21, Steareth-25 in CTFA nomenclature); addition products of ethylene oxide with isostearyl alcohol, comprising from 10 to 25 oxyethylene groups (for example Isosteareth-10, Isosteareth-12, Isosteareth-15, Isosteareth-20, Isosteareth-22, Isosteareth-25 in CTFA nomenclature); addition products of ethylene oxide with decyl alcohol, especially the product containing 10 oxyethylene groups (Deceth-10 in CTFA nomenclature); addition products of ethylene oxide with isocetyl alcohol, comprising from 10 to 25 oxyethylene groups (for example Isoceteth-10, Isoceteth-15, Isoceteth-20, Isoceteth-25); and mixtures thereof.

In one embodiment of the invention the oxyethylenated fatty alcohol is saturated.

In another embodiment of the invention the oxyethylenated fatty alcohol is linear.

According to one embodiment, use is made more particularly of the adducts of ethylene oxide with stearyl alcohol, especially those comprising from 10 to 25 oxyethylene groups (Steareth-10 to Steareth-25 in CTFA nomenclature), in particular having 10 oxyethylene groups.

The oxyethylenated nonionic surfactants having an ethylene oxide number of from 10 to 25 may be present in a total amount of from 0.1 wt% to 30 wt%, preferably from 0.5 wt% to 20 wt%, relative to the total weight of the reducing composition.

According to one preferred embodiment of the invention, the oxyethylenated nonionic surfactants having an ethylene oxide number of from 10 to 25 may be present in a total amount of greater than or equal to 1.5%, ranging preferably from 1.5 wt% to 30 wt%, more preferably from 1.5 % to 20 wt%, more preferably from 1.5 wt% to 10 wt%, relative to the total weight of the reducing composition.

According to one embodiment, the reducing composition comprises one or more thiol reducing agents as described above, one or more fatty substances solid at room temperature and selected from fatty alcohols in a total amount of greater than 4 wt% relative to the total weight of the composition, one or more cationic surfactants, ammonium bicarbonate, one or more oxyethylenated nonionic surfactants having an ethylene oxide unit number of from 10 to 25 selected from oxyethylenated fatty alcohols, the weight ratio of the fatty alcohols to the cationic surfactant(s) being less than or equal to 2.5, and with the proviso that the weight ratio of the total amount of fatty substance to the total amount of cationic surfactants present in the reducing composition is less than or equal to 2.5.

According to a further embodiment, the reducing composition comprises one or more thiol reducing agents as previously described, one or more fatty substances solid at room temperature and selected from fatty alcohols, fatty acid esters and/or fatty alcohol esters, mineral, vegetable or animal waxes, fatty amides, silicones, solid fatty ethers and mixtures thereof in a total amount of greater than 4 wt% relative to the total weight of the composition, one or more cationic surfactants selected from quaternary ammonium salts of formula (III), ammonium bicarbonate, one or more oxyethylenated nonionic surfactants having an ethylene oxide unit number of from 10 to 25 selected from oxyethylenated fatty alcohols, the weight ratio of said fatty substance or substances solid at room temperature and selected from fatty alcohols, fatty acid esters and/or fatty alcohol esters, mineral, vegetable or animal waxes, fatty amides, silicones, solid fatty ethers and mixtures thereof to said cationic surfactant(s) of formula (III) being less than or equal to 2.5, and with the proviso that the weight ratio of the total amount of fatty substance to the total amount of cationic surfactants present in the reducing composition is less than or equal to 2.5.

In accordance with this embodiment, the fatty substances are preferably selected from fatty alcohols.

According to yet another embodiment, the reducing composition comprises one or more thiol reducing agents as previously described, one or more fatty substances solid at room temperature and selected from fatty alcohols in a total amount of greater than 4 wt% less than or equal to 2.5, and with the proviso that the weight ratio of the total amount of fatty substance to the total amount of cationic surfactants present in the reducing composition is less than or equal to 2.5.

According to a further embodiment, the reducing composition comprises one or more thiol reducing agents as previously described, one or more fatty substances solid at room temperature and selected from fatty alcohols, fatty acid esters and/or fatty alcohol esters, mineral, vegetable or animal waxes, fatty amides, silicones, solid fatty ethers and mixtures thereof in a total amount of greater than 4 wt% relative to the total weight of the composition, one or more cationic surfactants selected from quaternary ammonium salts of formula (III), ammonium bicarbonate, one or more oxyethylenated nonionic surfactants having an ethylene oxide unit number of from 10 to 25, the weight ratio of said fatty substance or substances solid at room temperature and selected from fatty alcohols, fatty acid esters and/or fatty alcohol esters, mineral, vegetable or animal waxes, fatty amides, silicones, solid fatty ethers and mixtures thereof to said cationic surfactant(s) of formula (III) being less than or equal to 2.5, and with the proviso that the weight ratio of the total amount of fatty substance to the total amount of cationic surfactants present in the reducing composition is less than or equal to 2.5.

In accordance with this embodiment, the fatty substances are preferably selected from fatty alcohols.

According to yet another embodiment, the reducing composition comprises one or more thiol reducing agents as previously described, one or more fatty substances solid at room temperature and selected from fatty alcohols in a total amount of greater than 4 wt% relative to the total weight of the composition, one or more cationic surfactants selected from quaternary ammonium salts of formula (II), ammonium bicarbonate, one or more oxyethylenated nonionic surfactants having an ethylene oxide unit number of from 10 to 25 and selected from oxyethylenated fatty alcohols, especially those having a C₈-C₃₀ fatty chain, the weight ratio of said fatty alcohol or alcohols to said cationic surfactant(s) of formula (III) being less than or equal to 2.5, and with the proviso that the weight ratio of the total amount of fatty substance to the total amount of cationic surfactants present in the reducing composition is less than or equal to 2.5.

In accordance with this embodiment, the C₈-C₃₀ oxyethylenated fatty alcohols are selected from adducts of ethylene oxide with stearyl alcohol having from 10 to 25 oxyethylene groups.

According to the invention, the weight ratio of said fatty substance or substances solid at room temperature to said cationic surfactant or surfactants is preferably from 0.1 to 2.5, and more preferably from 1 to 2.5.

The reducing composition of the invention generally comprises water or a mixture of water and one or more organic solvents.

The suitable organic solvents include more particularly nonaromatic monoalcohols such as ethyl alcohol and isopropyl alcohol, or polyols or polyol ethers such as, for example, ethylene glycol monomethyl, monoethyl and monobutyl ethers, propylene glycol or its ethers such as, for example, propylene glycol monomethyl ether, butylene glycol, dipropylene glycol and also diethylene glycol alkyl ethers such as, for example, diethylene glycol monoethyl ether or monobutyl ether, or else polyols such as glycerol. Polyethylene glycols and polypropylene glycols, and mixtures of all these compounds, can also be used as solvent.

The above-described organic solvents, if present, represent commonly from 0.1 to 15 wt%, more preferably from 0.5 to 5 wt%, relative to the total weight of the composition.

The pH of the reducing composition according to the invention is generally from 7 to 10 and preferably from 7.5 to 9.5. It may be adjusted by addition either of alkaline agents such as aqueous ammonia, monoethanolamine, diethanolamine, triethanolamine, isopropanolamine, 2-methyl-2-amino-1-propanol, propane-1,3-diamine, guanidine, arginine, an ammonium or alkali metal carbonate or bicarbonate, an organic carbonate such as guanidine carbonate, or else an alkali metal hydroxide, or of acidifying agents such as hydrochloric acid, acetic acid, lactic acid, boric acid, citric acid and phosphoric acid.

The composition according to the invention preferably comprises at least one alkaline agent.

The reducing composition may more particularly comprise aqueous ammonia or one or more alkanolamines, especially monoethanolamine.

As mentioned above, the composition according to the invention comprises ammonium bicarbonate.

The cosmetic composition in accordance with the invention may further comprise one or more additional cosmetic agents.

The additional cosmetic agent or agents may be selected from anionic, cationic, nonionic, amphoteric and zwitterionic polymers or mixtures thereof; anionic surfactants, nonionic surfactants different from the oxyethylenated nonionic surfactants of the invention, or amphoteric surfactants; pigments; thickeners; antioxidants; penetrants; sequestrants; fragrances; buffers; dispersants; reduction regulators such as dithio acids, such as dithiodiglycolic acid and salts thereof; film-formers; preservatives; stabilizers; opacifiers; and fragrances.

Of course, those skilled in the art will take care to select this or these optional additional compound(s) such that the advantageous properties intrinsically associated with the composition in accordance with the invention are not, or are not substantially, adversely affected by the envisaged addition(s).

The above additional cosmetic agents are generally present in an amount, for each of them, of between 0 and 20 wt%, relative to the total weight of the composition.

The reducing composition may preferably comprise one or more thickeners, which may be selected from associative or non-associative, nonionic or cationic, zwitterionic, amphoteric, or anionic, natural or synthetic polymeric thickeners, and from non-polymeric thickeners such as, for example, an electrolyte or a sugar.

Polymeric thickeners include, for example, cellulosic thickeners, for example hydroxyethyl cellulose, hydroxypropyl cellulose and carboxymethyl cellulose, guar gum and its derivatives, for example hydroxypropyl guar, sold by the company Rhodia under the name Jaguar HP 105, gums of microbial origin, such as xanthan gum and scleroglucan gum, synthetic polymeric thickeners such as crosslinked homopolymers of acrylic acid or of acrylamidopropanesulfonic acid, for example Carbomer, nonionic, anionic or amphoteric associative polymers, such as the polymers sold under the names Pemulen TR1 or TR2 by the company Goodrich, Salcare SC90 by the company Allied Colloids, Aculyn 22, 28, 33, 44 or 46 by the company Rohm & Haas, and Elfacos T210 and T212 by the company Akzo.

The cosmetic composition preferably comprises one or more additional thickeners selected from nonionic thickeners, more particularly polysaccharides.

The reducing composition is preferably present in the form of a cream.

The reducing composition according to the invention may advantageously have a viscosity at 25°C of from 2 to 20 Pa.s, more preferably of from 2 to 15 Pa.s and more preferably still of from 2.5 to 10 Pa.s.

The viscosity of the reducing composition is determined at 25°C by means of a Rheomat 180 rotary viscometer (Jean Lamy SA) equipped with a spindle 3 (MS r3) or spindle 4 (MS r4) anchor/bucket geometer with a rotary speed of 200 revolutions/minute, with use of the calibration plots supplied by the viscometer supplier.

The present invention likewise relates to a method for permanent deformation treatment of keratinous fibres, more particularly of human keratinous fibres such as the hair, which comprises performing the following steps:
(i) a reducing composition according to the invention is applied to said fibres, and the time sufficient for shaping is allowed to elapse, and
(ii) optionally, an oxidizing composition is applied to said fibres for a time sufficient to fix the shape.

The waiting time allowed to elapse following application of the reducing composition is generally from 5 to 60 minutes, in particular from 3 to 30 minutes and preferably from 5 to 15 minutes.

The oxidizing composition used in step (ii) of the permanent deformation method conventionally comprises one or more oxidizing agents, in general aqueous hydrogen peroxide solution, an alkali metal bromate, a persalt or a polythionate, and even more preferentially aqueous hydrogen peroxide solution.

The pH of the oxidizing composition is generally from 2 to 10.

The waiting time for the oxidizing composition is generally from 2 to 30 minutes, preferably from 5 to 15 minutes.

In particular, the reducing composition according to the invention is applied in order to reduce the disulfide links in the keratin, with the keratinous fibres being placed under mechanical tension before, during or after said application.

When a perming operation is desired, preference is given to using mechanical means such as curlers in order to place the keratinous fibres under tension, with the reducing composition according to the invention being applied before, during or after the hair-shaping means, preferably after.

The reducing composition in accordance with the present invention may be applied to wetted hair which has been wound beforehand onto rollers with a diameter of from 2 to 30 mm. The reducing composition may also be applied in line with the winding of the hair. Generally speaking, the reducing composition is then allowed to act for a time of 5 to 60 minutes.

Following application of the reducing composition according to the invention, it is also possible to subject the whole hair to a heat treatment by heating at a temperature of between 30 and 250°C for all or part of the leave-on time. In practice, this operation may be performed using a hairstyling hood, a hairdryer, a round or flat iron, an infrared ray dispenser and other heating appliances.

In particular it is possible, both as heating means and as hair-shaping means, to use heating tongs at a temperature of between 60 and 230°C, and preferably between 120 and 230°C, the heating tongs being used after the step of interim rinsing following the application of the reducing composition.

The curler itself may be a heating means.

The oxidizing composition for reforming the keratin disulfide bonds is then applied to the rolled-up or unrolled hair, generally for a leave-on time of from 2 to 15 minutes.

In the case of a hair relaxing or straightening process, the reducing composition is applied to the hair, and the hair is then subjected to mechanical deformation for fixing the hair in its new shape, by means of a hair straightening operation, with a wide-toothed comb, with the back of a comb, by hand or with a brush. The leave-on time is generally from 5 to 60 minutes.

This application may also be followed with a heating treatment, especially using an iron.

The straightening of the hair may also be performed, totally or partly, using a heating iron at between 60 and 230°C and preferably between 120 and 230°C.

The oxidizing composition as defined above is then applied, and is left to act generally for around 2 to 15 minutes, after which the hair is optionally rinsed thoroughly, generally with water.

After the permanent deformation treatment method has been performed, the keratinous fibres are optionally rinsed.

Preferably, the keratinous fibres impregnated with the oxidizing composition are rinsed thoroughly with water. The keratinous fibres may optionally be separated, before or after, from the means needed to keep them under tension.

The keratinous fibres may then be washed with a shampoo, rinsed and dried or left to dry.

The permanent deformation treatment method is preferably a method for smoothing of keratinous fibres, more particularly of human keratinous fibres such as the hair.

The example that follows serves to illustrate the invention without, however, being limiting in nature.

### EXAMPLE

### I. Reducing compositions

The reducing compositions (A), (B) and (C) are prepared from the ingredients indicated as a weight percentage of commercial product as it is in the table below:

| | Composition A (Comparative) | Composition B (Invention) | Composition C (Invention) |
|---|---|---|---|
| Cationic surfactant ⁽¹⁾ | 16.88% | 16.88% | 16.88% |
| Cetylstearyl alcohol ⁽²⁾ | 10% | 10% | 5% |
| Steareth-20 ⁽³⁾ | - | 4.2% | 2% |
| Ethoxylated cetyl alcohol ⁽⁴⁾ | 3% | - | - |
| Ammonium thioglycolate at 71% in aqueous solution ⁽⁵⁾ | 8.5% | 8.5% | 8.5% |
| Ammonium bicarbonate | 2.5% | 2.5% | 2.5% |
| Monoethanolamine | qs pH = 8.5 | qs pH = 8.5 | qs pH = 8.5 |
| Pentasodium pentetate at 40% in aqueous solution ⁽⁶⁾ | 0.2% | 0.2% | 0.2% |
| Fragrance | qs | qs | qs |
| Water | qs 100 | qs 100 | qs 100 |

| | | | |
|---|---|---|---|
| ⁽¹⁾ Cetyltrimethylammonium chloride at 25% in aqueous solution, sold under the name Genamin CTAC 25 by Clariant ⁽²⁾ Fatty alcohol sold under the name Ecorol 68/50P by Ecogreen Oleochemicals ⁽³⁾ Nonionic surfactant sold under the name Tego Alkanol S20P by Evonik Goldschmidt ⁽⁴⁾ Nonionic surfactant sold under the name Brij C2-SO-(AP) by Croda ⁽⁵⁾ Reducing agent sold under the name Bruno Bock ⁽⁶⁾ Chelating agent sold under the name Versenex 80 by Dow Chemical | | | |

### II. Physicochemical properties

The viscosity of reducing compositions (A), (B) and (C) is measured at 25°C using a Rheomat 180 rotary viscometer equipped with a spindle 3 (MS r3) or spindle 4 (MS r4) anchor/bucket geometer. The value read at the rotary speed of 200 revolutions/minute and after a time of 10 minutes is expressed in deviation units (DU) and converted to Cps (mPa.s) on the basis of supplier data.

| | Composition A (Comparative) | Composition B (Invention) | Composition C (Invention) |
|---|---|---|---|
| Final viscosity | 1060 | 7130 | 2620 |

The comparative composition (A) is found to have a low final viscosity. It is also found that composition (A), before the addition of the active reducing agents and alkaline agents, has a high viscosity (greater than or equal to 4000 cps) that collapses on the addition of the active reducing agents and alkaline agents, to give a viscosity of 1060 cps at the end of manufacture.

Moreover, the comparative composition (A) is present in the form of a fluid cream with a texture which is unstable over time.

The inventive reducing compositions (B) and (C) take the form of creams which are smooth and uniform throughout manufacture, with no change in their texture during storage.

### III. Method

The reducing compositions (B) and (C) are applied to hair which has been washed beforehand and then wrung out, over the entirety of the hair.

After a waiting time of 30 minutes, the hair is rinsed with water. The oxidizing composition "Fixateur X Tenso Moisturist" from L'Oréal is then applied to the whole of the hair. After a waiting time of 10 minutes, the hair is rinsed.

The hair is observed to have a very smooth appearance and a very pleasant feel.

## Claims

1. Reducing cosmetic composition comprising:
- one or more sulfur-containing reducing agents selected from thioglycolic acid, thiolactic acid, thiomalic acid, 2-mercaptopropionic acid, 3-mercaptopropionic acid, thiosalicylic acid, thiopropionic acid, and salts thereof,
- one or more fatty substances solid at room temperature and present in a total amount of greater than or equal to 4 wt%, relative to the total weight of the composition,
- one or more cationic surfactants, and
- one or more oxyethylenated nonionic surfactants having an ethylene oxide unit number of from 10 to 25 selected from oxyethylenated saturated fatty alcohols, with the weight ratio of the total amount of solid fatty substances to the total amount of cationic surfactant(s) being less than or equal to 2.5, and
- ammonium bicarbonate.

2. Reducing composition according to Claim 1, **characterized in that** the sulfur-containing reducing agents are selected from thioglycolic acid and thiolactic acid or salts thereof, especially alkali metal, alkaline-earth metal or ammonium salts, and mixtures thereof.

3. Reducing composition according to Claims 1 and 2, **characterized in that** the fatty substances solid at room temperature are selected from fatty alcohols, fatty acid esters and/or fatty alcohol esters, mineral, vegetable or animal waxes, fatty amides, silicones, solid fatty ethers and mixtures thereof.

4. Reducing composition according to any of the preceding claims, **characterized in that** the fatty substances are selected from fatty alcohols, esters of C₉-C₂₆ fatty acids and C₉-C₂₆ fatty alcohols and mixtures thereof.

5. Reducing composition according to any of the preceding claims, **characterized in that** the fatty substances are selected from saturated linear fatty alcohols comprising from 12 to 30 carbon atoms and more particularly from cetyl alcohol, stearyl alcohol and mixtures thereof.

6. Reducing composition according to any of the preceding claims, **characterized in that** the cationic surfactants are selected from salts of optionally polyoxyalkylenated primary, secondary or tertiary fatty amines, quaternary ammonium salts, and mixtures thereof.

7. Reducing composition according to Claim 6, **characterized in that** the cationic surfactants are selected from:
- quaternary ammonium salts corresponding to the general formula (III) below: 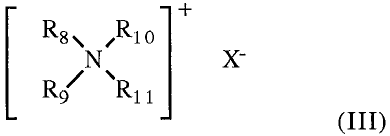 in which the radicals R₈ to R₁₁, which may be identical or different, represent a linear or branched aliphatic radical comprising from 1 to 30 carbon atoms, or an aromatic radical such as aryl or alkylaryl, with at least one of the radicals R₈ to R₁₁ denoting a linear or branched alkyl radical comprising from 10 to 30 carbon atoms, and X⁻ denoting an organic or inorganic anion,
- quaternary ammonium salts of imidazoline,
- quaternary di- or triammonium salts, more particularly of formula (V) below: 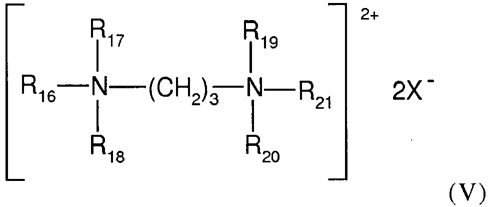 in which formula (V):
R₁₆ denotes an alkyl group comprising approximately from 16 to 30 carbon atoms, which is optionally hydroxylated and/or interrupted with one or more oxygen atoms;
R₁₇ is selected from hydrogen, an alkyl group comprising from 1 to 4 carbon atoms or a group -(CH₂)₃-N+(R₁₆ₐ)(R₁₇ₐ)(R₁₈ₐ), X⁻;
R₁₆ₐ, R₁₇ₐ, R₁₈ₐ, R₁₈, R₁₉, R₂₀ and R₂₁, which may be identical or different, are selected from hydrogen and an alkyl group comprising from 1 to 4 carbon atoms; and
X⁻, which are identical or different, represent an organic or inorganic anionic counterion, such as that selected from halides, acetates, phosphates, nitrates, alkyl(C₁-C₄) sulfates, alkyl(C₁-C₄)- or alkyl(C₁-C₄)aryl-sulfonates, more particularly methyl sulfate and ethyl sulfate;
- quaternary ammonium salts containing at least one ester function.

8. Reducing composition according to Claim 7, **characterized in that** the cationic surfactants are selected from quaternary ammonium salts of formula (III).

9. Reducing composition according to any of the preceding claims, **characterized in that** the weight ratio of the total amount of fatty substance(s) solid at room temperature to the total amount of cationic surfactant(s) is from 0.1 to 2.5 and more preferably from 1 to 2.5.

10. Reducing composition according to any of the preceding claims, **characterized in that** the oxyethylenated nonionic surfactants are selected from oxyethylenated, linear or branched, saturated, C₈-C₃₀, preferably C₁₂-C₂₂, fatty alcohols,.

11. Reducing composition according to any of the preceding claims, **characterized in that** the nonionic surfactants are selected from C₈-C₃₀ oxyethylenated fatty alcohols having an ethylene oxide number of 10 to 25, more particularly the addition products of ethylene oxide with stearyl alcohol containing from 10 to 25 oxyethylene groups.

12. Reducing composition according to any of the preceding claims, **characterized in that** the nonionic surfactants having an ethylene oxide number of from 10 to 25 are present in a total amount of 0.1 wt% to 30 wt%, more preferably from 0.5 wt% to 20 wt%, relative to the total weight of the reducing composition.

13. Method for permanent deformation treatment, especially smoothing and permanent waving, preferably smoothing, of keratinous fibres, more particularly of human keratinous fibres such as the hair, which comprises performing the following steps:
(i) a reducing composition as defined in any of claims 1 to 12 is applied to said fibres, and the time sufficient for shaping is allowed to elapse, and
(ii) optionally, an oxidizing composition is applied to said fibres for a time sufficient to fix the shape.

## Patentansprüche

1. Reduzierende kosmetische Zusammensetzung, umfassend:
- ein oder mehrere schwefelhaltige Reduktionsmittel, die aus Thioglykolsäure, Thiomilchsäure, Thioäpfelsäure, 2-Mercaptopropionsäure, 3-Mercaptopropionsäure, Thiosalicylsäure, Thiopropionsäure und Salzen davon ausgewählt sind,
- eine oder mehrere Fettsubstanzen, die bei Raumtemperatur fest sind und in einer Gesamtmenge größer oder gleich 4 Gew.-%, bezogen auf das Gesamtgewicht der Zusammensetzung, vorliegen,
- ein oder mehrere kationische Tenside und
- ein oder mehrere oxyethylenierte nichtionische Tenside mit einer Zahl von Ethylenoxid-Einheiten von 10 bis 25 aus oxyethylenierten gesättigten Fettalkoholen davon ausgewählt sind, wobei das Gewichtsverhältnis der Gesamtmenge an festen Fettsubstanzen zur Gesamtmenge an kationischem Tensid bzw. kationischen Tensiden kleiner oder gleich 2,5 ist, und
- Ammoniumhydrogencarbonat.

2. Reduzierende Zusammensetzung nach Anspruch 1, **dadurch gekennzeichnet, dass** die schwefelhaltigen Reduktionsmittel aus Thioglykolsäure und Thiomilchsäure oder Salzen davon, insbesondere Alkalimetall-, Erdalkalimetall- oder Ammoniumsalzen, und Mischungen davon ausgewählt sind.

3. Reduzierende Zusammensetzung nach den Ansprüchen 1 und 2, **dadurch gekennzeichnet, dass** die Fettsubstanzen, die bei Raumtemperatur fest sind, aus Fettalkoholen, Fettsäureestern und/oder Fettalkoholestern, mineralischen, pflanzlichen oder tierischen Wachsen, Fettamiden, Silikonen, festen Fettethern und Mischungen davon ausgewählt sind.

4. Reduzierende Zusammensetzung nach einem der vorhergehenden Ansprüche, **dadurch gekennzeichnet, dass** die Fettsubstanzen aus Fettalkoholen, Estern von C₉-C₂₆-Fettsäuren und C₉-C₂₆-Fettalkoholen und Mischungen davon ausgewählt sind.

5. Reduzierende Zusammensetzung nach einem der vorhergehenden Ansprüche, **dadurch gekennzeichnet, dass** die Fettsubstanzen aus gesättigten linearen Fettalkoholen mit 12 bis 30 Kohlenstoffatomen und spezieller aus Cetylalkohol, Stearylalkohol und Mischungen davon ausgewählt sind.

6. Reduzierende Zusammensetzung nach einem der vorhergehenden Ansprüche, **dadurch gekennzeichnet, dass** die kationischen Tenside aus Salzen von gegebenenfalls polyoxyalkylenierten primären, sekundären oder tertiären Fettaminen, quaternären Ammoniumsalzen und Mischungen davon ausgewählt sind.

7. Reduzierende Zusammensetzung nach Anspruch 6, **dadurch gekennzeichnet, dass** die kationischen Tenside aus:
- quaternären Ammoniumsalzen der nachstehenden allgemeinen Formel (III): 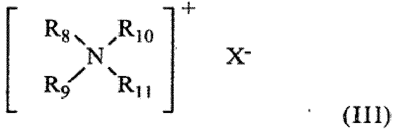 wobei die Reste R₈ bis R₁₁, die gleich oder verschieden sein können, für einen linearen oder verzweigten aliphatischen Rest mit 1 bis 30 Kohlenstoffatomen oder einen aromatischen Rest wie Aryl oder Alkylaryl stehen, wobei mindestens einer der Reste R₈ bis R₁₁ einen linearen oder verzweigten Alkylrest mit 10 bis 30 Kohlenstoffatomen darstellt, und X⁻ für ein organisches oder anorganisches Anion steht,
- quaternären Ammoniumsalzen von Imidazolin,
- quaternären Di- oder Triammoniumsalzen, spezieller der nachstehenden Formel (V): 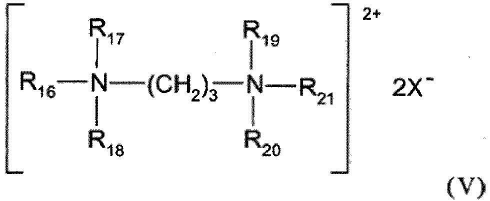 wobei in der Formel (V):
R₁₆ für eine Alkylgruppe mit ungefähr 16 bis 30 Kohlenstoffatomen, die gegebenenfalls hydroxyliert und/oder durch ein oder mehrere Sauerstoffatome unterbrochen ist, steht;
R₁₇ aus Wasserstoff, einer Alkylgruppe mit 1 bis 4 Kohlenstoffatomen oder einer Gruppe -(CH₂)₃-N+(R₁₆ₐ)(R₁₇ₐ)(R₁₈ₐ), X⁻ ausgewählt ist;
R₁₆ₐ, R₁₇ₐ, R₁₈ₐ, R₁₈, R₁₉, R₂₀ und R₂₁, die gleich oder verschieden sein können, aus Wasserstoff und einer Alkylgruppe mit 1 bis 4 Kohlenstoffatomen ausgewählt sind; und
X⁻, die gleich oder verschieden sein können, für ein organisches oder anorganisches anionisches Gegenion, wie dasjenige, das aus Halogeniden, Acetaten, Phosphaten, Nitraten, Alkyl (C₁-C₄)-sulfaten, Alkyl (C₁-C₄)- oder Alkyl (C₁-C₄)-arylsulfonaten, spezieller Methylsulfat und Ethylsulfat, ausgewählt ist, stehen;
- quaternären Ammoniumsalzen mit mindestens einer Esterfunktion
ausgewählt sind.

8. Reduzierende Zusammensetzung nach Anspruch 7, **dadurch gekennzeichnet, dass** die kationischen Tenside aus quaternären Ammoniumsalzen der Formel (III) ausgewählt sind.

9. Reduzierende Zusammensetzung nach einem der vorhergehenden Ansprüche, **dadurch gekennzeichnet, dass** das Gewichtsverhältnis der Gesamtmenge an Fettsubstanzen, die bei Raumtemperatur fest sind, zur Gesamtmenge an kationischem Tensid bzw. kationischen Tensiden 0,1 bis 2,5 und weiter bevorzugt 1 bis 2,5 beträgt.

10. Reduzierende Zusammensetzung nach einem der vorhergehenden Ansprüche, **dadurch gekennzeichnet, dass** die oxyethylenierten nichtionischen Tenside aus oxyethylenierten, linearen oder verzweigten, gesättigten C₈-C₃₀-Fettalkoholen, vorzugsweise C₁₂-C₂₂-Fettalkoholen, davon ausgewählt sind.

11. Reduzierende Zusammensetzung nach einem der vorhergehenden Ansprüche, **dadurch gekennzeichnet, dass** die nichtionischen Tenside aus oxyethylenierten C₈-C₃₀-Fettalkoholen mit einer Ethylenoxid-Zahl von 10 bis 25, spezieller den Additionsprodukten von Ethylenoxid mit Stearylalkohol mit 10 bis 25 Oxyethylen-Gruppen, ausgewählt sind.

12. Reduzierende Zusammensetzung nach einem der vorhergehenden Ansprüche, **dadurch gekennzeichnet, dass** die nichtionischen Tenside mit einer Ethylenoxid-Zahl von 10 bis 25 in einer Gesamtmenge von 0,1 Gew.-% bis 30 Gew.-%, weiter bevorzugt von 0,5 Gew.-% bis 20 Gew.-%, bezogen auf das Gesamtgewicht der reduzierenden Zusammensetzung, vorliegen.

13. Verfahren zur Dauerverformungsbehandlung, insbesondere Glättung und Dauerwellung, vorzugsweise Glättung, von Keratinfasern, spezieller von menschlichen Keratinfasern wie dem Haar, das die Durchführung der folgenden Schritte umfasst:
(i) eine reduzierende Zusammensetzung gemäß einem der Ansprüche 1 bis 12 wird auf die Fasern aufgebracht, und der für die Formgebung ausreichende Zeitraum wird verstreichen gelassen, und
(ii) gegebenenfalls wird eine oxidierende Zusammensetzung über einen zur Fixierung der Form ausreichenden Zeitraum auf die Fasern aufgebracht.

## Revendications

1. Composition cosmétique réductrice comprenant :
- un ou plusieurs agents réducteurs soufrés choisis parmi l'acide thioglycolique, l'acide thiolactique, l'acide thiomalique, l'acide 2-mercaptopropionique, l'acide 3-mercaptopropionique, l'acide thiosalicylique, l'acide thiopropionique, et leurs sels,
- un ou plusieurs corps gras solides à température ambiante ayant une teneur totale supérieure ou égale à 4% en poids par rapport au poids total de la composition,
- un ou plusieurs tensioactifs cationiques et,
- un ou plusieurs tensioactifs non ioniques oxyéthylénés ayant un nombre d'unité d'oxyde d'éthylène allant de 10 à 25 choisis parmi les alcools gras saturés oxyéthylénés ; le rapport pondéral entre la teneur totale de corps gras solides et la teneur totale de tensioactif(s) cationique(s) étant inférieur ou égal à 2,5 et
- du bicarbonate d'ammonium.

2. Composition réductrice selon la revendication 1, **caractérisée en ce que** les agents réducteurs soufrés sont choisis parmi l'acide thioglycolique, l'acide thiolactique ainsi que leurs sels, notamment de métaux alcalin, alcalino-terreux ou d'ammonium, et leurs mélanges.

3. Composition réductrice selon la revendication 1 ou 2, **caractérisée en ce que** les corps gras solides à température ambiante sont choisis parmi les alcools gras, les esters d'acide gras et/ou d'alcool gras, les cires minérales, végétales ou animales, les amides gras, les silicones, les éthers gras, solides ainsi que leurs mélanges.

4. Composition réductrice selon l'une quelconque des revendications précédentes, **caractérisée en ce que** les corps gras sont choisis parmi les alcools gras, les esters d'acides gras en C₉-C₂₆ et d'alcools gras en C₉-C₂₆ et leurs mélanges.

5. Composition réductrice selon l'une quelconque des revendications précédentes, **caractérisée en ce que** les corps gras sont choisis parmi les alcools gras saturés linéaires, comportant de 12 à 30 atomes de carbone et en particulier parmi l'alcool cétylique, l'alcool stéarylique et leurs mélanges.

6. Composition réductrice selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire, et leurs mélanges.

7. Composition réductrice selon la revendication 6, **caractérisée en ce que** les tensioactifs cationiques sont choisis parmi :
- les sels d'ammonium quaternaire répondant à la formule générale (III) suivante : 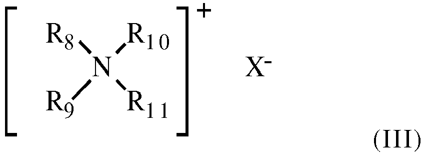 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle au moins un des radicaux R₈ à R₁₁ désignant un radical alkyle, linéaire ou ramifié, comportant de 10 à 30 atomes de carbone et X- désignant un anion minéral ou organique,
- les sels d'ammonium quaternaire de l'imidazoline,
- les sels de di- ou de triammonium quaternaire en particulier de formule (V) suivante : 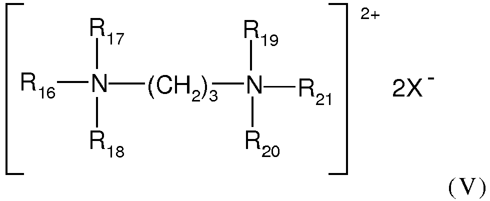 Formule (V) dans laquelle :
R₁₆ désigne un groupe alkyle comportant environ de 16 à 30 atomes de carbone, éventuellement hydroxylé et/ou interrompu par un ou plusieurs atomes d'oxygène ;
R₁₇ est choisi parmi l'hydrogène, un groupe alkyle comportant de 1 à 4 atomes de carbone ou un groupe -(CH₂)₃-N+(R₁₆ₐ)(R₁₇ₐ)(R₁₈ₐ), X⁻ ;
R₁₆ₐ, R₁₇ₐ, R₁₈ₐ, R₁₈, R₁₉, R₂₀ et R₂₁, identiques ou différents, sont choisis parmi l'hydrogène et un groupe alkyle comportant de 1 à 4 atomes de carbone ; et
X⁻, identiques ou différents, représentent un contre ion anionique organique ou inorganique, tels que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate ;
- les sels d'ammonium quaternaire contenant au moins une fonction ester.

8. Composition réductrice selon la revendication 7, **caractérisée en ce que** les tensioactifs cationiques sont choisis parmi les sels d'ammonium quaternaire de formule (III).

9. Composition réductrice selon l'une quelconque des revendications précédentes, **caractérisée en ce que** le rapport pondéral entre la quantité totale de corps gras solide(s) à température ambiante et la quantité totale de tensioactif(s) cationique(s) varie de 0,1 à 2,5 et plus préférentiellement de 1 à 2,5.

10. Composition réductrice selon l'une quelconque des revendications précédentes, **caractérisée en ce que** les tensioactifs non ioniques oxyéthylénés sont choisis parmi les alcools gras en C₈-C₃₀, de préférence en C₁₂-C₂₂, saturés, linéaires ou ramifiés, oxyéthylénés.

11. Composition réductrice selon l'une quelconque des revendications précédentes, **caractérisée en ce que** les tensioactifs non ioniques sont choisis parmi les alcools gras oxyéthylénés en C₈-C₃₀ ayant un nombre d'oxyde éthylène allant de 10 à 25, en particulier les produits d'addition d'oxyde d'éthylène avec l'alcool stéarylique comportant de 10 à 25 groupes oxyéthylène.

12. Composition réductrice selon l'une quelconque des revendications précédentes, **caractérisée en ce que** les tensioactifs non ioniques ayant un nombre d'oxyde éthylène allant de 10 à 25 sont présents dans une teneur totale allant de 0,1% à 30% en poids, mieux de 0,5 à 20% en poids par rapport au poids total de la composition réductrice.

13. Procédé de traitement de déformation permanente, notamment de lissage et de permanente, de préférence de lissage, des fibres kératiniques, en particulier des fibres kératiniques humaines telles que les cheveux, consistant à mettre en oeuvre les étapes suivantes :
(i) on applique sur lesdites fibres une composition réductrice telle que définie selon l'une quelconque des revendications 1 à 12, on laisse pauser pendant la durée suffisante à la mise en forme, et
(ii) éventuellement, on applique sur lesdites fibres une composition oxydante pendant une durée suffisante à la fixation de la forme.
